# NEW EUROPEAN PATENT SPECIFICATION

(11) **EP 0 514 452 B2**
(45) Date of publication and mention of the opposition decision: **19.07.2000**
(45) Mention of the grant of the patent: 18.08.1993
(21) Application number: 91904163.2
(22) Date of filing: 23.01.1991
(51) Int. Cl.: A61K 31/425, A61K 31/44, A61K 31/155, A61K 31/40, A61K 31/235, A61K 31/135, A61K 31/35

(54) **USE OF INSULIN SENSITIZING AGENTS TO TREAT HYPERTENSION**
VERWENDUNG INSULIN-SENSIBLISIERENDER WIRKSTOFFE ZUR BEHANDLUNG VON HYPERTONIE
UTILISATION D'AGENTS DE SENSIBILISATION A L'INSULINE AFIN DE TRAITER L'HYPERTENSION

(30) Priority: 09.02.1990 US 478090
(43) Date of publication of application: 25.11.1992
(73) Proprietor: THE UPJOHN COMPANY, Kalamazoo, Michigan 49001 (US)
(72) Inventor: COLCA, Jerry, R., Kalamazoo, MI 49009 (US)
(74) Representative: Perry, Robert Edward
(86) International application number: US9100348
(87) International publication number: WO9112003

(56) References cited:
- EP-A- 0 006 735
- EP-A- 0 008 203
- EP-A- 0 142 102
- EP-A- 0 146 392
- EP-A- 0 166 183
- EP-A- 0 193 256
- EP-A- 0 283 369
- EP-A- 0 356 214
- GB-A- 2 188 234
- US-A- 3 542 927
- US-A- 4 287 200
- US-A- 4 382 958
- US-A- 4 572 912
- US-A- 4 687 777
- The New England J. of Medicine, vol. 317, 1987, Melbourne E. Ferrannini et al., pp. 350-357
- Biochemical and Biophysical Research Communications, vol. 128, 1985, R.A.J. Chaliss et al., pp. 928-935
- Diabetic Medicine, vol. 6, 1989, O. Pedersen et al., pp. 249-256
- Diabetes, vol. 35, suppl. 1, 1986, Anaheim, US, p. 66 A, M.V. Sennitt et al., abstract no. 262 and M.A. Cawthorne et al., abstract no. 263
- Diabetes, vol. 32, 1983, A.Y. Chang et al., pp. 835-845
- The J. of Clinical Investigation, vol. 75, 1985, M. Modan et al., pp. 809-817
- Pered et al., Am. J. Physiol., 202(2), 249-252, (1962)
- Bravo et al., Am. J. Hypertension, 2, (1989), 339S-344S
- Shen et al., J. Clin. Endocrinology and Medicine, 66, (1988), 580-583
- Zavaroni et al., New England Journal of Medicine, 320(11), (1989), 702-706
- Reaven et al, Lancet, 2, (1984), 435-436
- Krotkiewski et al., Metabolism, 28(6), (1979), 650-659
- Reaven et al, Hypertension, 12(2), (1988), 129-133
- Carretta et al., Hypertension, 7(6), (1989), S196-S197
- Lucas et al., Hypertension, 7, (1985), 702-706
- Manicardi et al., J. Clin. Endocrinol. Metab., 62, (1986), 1302-1304
- Reaven et al., Hypertension, 14, (1989), 117-120
- Tedde et al., Am. J. Hypertension, 2, (1989), 163-170
- Landsberg, Yale Journal of Biology and Medicine, 62, (1989), 511-519
- Rose et al., Hypertension, 8, (1986), 793-800
- Berglund et al., Hypertension, 4, (1982), 692-696
- MacMahan et al., Lancet, (1985), 1233-1236
- Sowers et al., Hypertension, 4, (1982) 686-691
- Rocchini et al., Hypertension, 13, (1989), 922-928
- Krieger and Landsberg, Am. J. Hypertension, 1, (1988), 84-90
- Connacher et al., Br. Med. J., 296, (1988), 1217-1220
- Rochet et al., Am. J. Physion (Endocrinol. Metab. 18), E101-E109, (1988)
- Anderson et al., Am. J. Gastroenterol., 81, (1986), 907-919
- Jimenez et al., J. Clin. Endocrinol. Metab., 64. (1987), 661-668
- Kurtz et al., Hypertension, 13, (1989), 896-901
- Reaven et al., Lancet, (1987), 435-436
- Kaplan et al., Arch. Intern. Med., 149, (1989), 1514-1520
- Landsberg et al., Quarterly J. Medicine, New Series 01. No 230, 1081-1090, December 1980
- Hedstrand H., A study of middle-aged men with particular reference to risk factors for cardiovascular disease. Thesis. Upsala J Med Sci 1975; suppl 19
- Skarfors ET, Lithell HO, Eclinue I, Aberg H, Do antihyportensive drugs precipitate diabetes in predisposed men? Br Med J 1989; 298:1147-1152
- Skarfors ET, Sellnus KI, Lithell HO, Risk factors for development of non-inulin dependent diabetes in middle-aged men, Results from a 10 year follow-up of participants in an Uppsala health survey, Brit Med J 1991:303; 755-760
- Pollare T, Lithell H, Selinus I, Berne C, Sensitivity to insulin during treatment with atenolol and metopropol a randomised, double blind study of effect on carbohydtrate and lipoprotein netabolism in hypertensive patients, Br Med J 1989; 298:1152-1157
- Pollare T, Lithell H, Morlin C, Prantare H, Hvarfner A, Ljunghall S, Matabolic effects of diltiazem and atenolol: results from a randomised, double-blind study with parallel groups, J Hypertens 1989; 7:551-559
- Pollare T, Lithell H, Selinus K, Berne C, Application of prazosin is associated with an increase of insulin sensitivity in obese patients with hypertension. Diabetologia 1988; 31:415-420
- Pollare T, Lithell H, Berne C, Insulin resistance is a chracteristic feature of primary hypertension independentiy of obesitv. Metabolism 1990; 39:167-174
- Anderson PE, Johansson J, Berne C, Lithell H, Effects of selective alfa1 and beta1-adreno-receptor blockade on lipoprotein and carbohydrate metabolism in hypertensive subjects, with special emphasis on insulin sensitivity, Journal of Human Hypertension (1994) 8, 219-226
- Skarfors ET, Lithell HO, Selinus I, Risk factors for development of hypertension: a 10-year longitudinal study in middle-aged men, J Hypertens, 1991; 9:217-223
- Pollare T, Lithell H, Berne C, A comparison of the effects of hydrochlorothiazide and captoril on glucose and lipid metabolism in patients with hypertension, N Engl J Med. 1989; 321:868-873
- Haenni A, Lithell H, Treatment with a B-blocker with B2-Aonism Improves Glucose and lipid Metabolism in essential hypertension. Metabolism vol 43, No. 4 (April), 1994, pp. 455-461
- Reaven GM, Banting lecture 1988, Role of insulin resistance in human disease, Diabetes 1988; 37:1595-1607
- Chang et al., Diabetes, 32, (1983), 830-838

## Description

The present invention provides a new use of known pharmaceutical compounds. In particular, the present invention provides for the treatment of hypertension with certain insulin sensitizing agents such as thiazolidinedione derivatives. These compounds are previously known for the treatment of diabetes.

The fact that there was a relationship between circulating insulin and hypertension has been frequently discussed in the literature. Thus, for example, Pereda, et al, Am. J. Physiol. 202 (2): 249-252 (1962) noted an increase in blood pressure in dogs due to the administration of insulin. DeFronzo, Diabetologia 21: 165-171 (1981) attributed this increase in hypertension to the effect of insulin on renal sodium retention which expanded the vascular volume, while Rowe, et al, Diabetes 30:219-225 (March 1981) attributed it to the increased activity of the sympathetic nervous system. Other studies have suggested that hyperinsulinemia as the result of insulin resistance is associated with hypertension. This is attributed to the fact that obesity is known to be associated with insulin resistance and it is a commonly held view that hyperinsulinemia in obesity is a major factor responsible for hypertension. See, e.g., Modan, et al, J. Clin. Invest. 75:809-817 (March 1985). Patients with essential hypertension have been reported to have insulin resistance. Ferrannini, et al, N. Eng. J. Med. 317:350-7 (1987). In the last study a measure of insulin resistance was reported to directly correlate with arterial blood pressure. In patients with a functional endocrine pancreas, insulin resistance also correlates directly with circulating insulin levels.

Ciglitazone is characteristic of a new class of thiazolidine antidiabetic agents which lower blood glucose in animal models of noninsulin diabetes mellitus (NIDDM), while actually reducing circulating concentrations of insulin. This is believed to be accomplished by improving the responsiveness of the peripheral tissues to insulin. See, e.g., Chang, et al, Diabetes 32:830-838 (September 1983).

Because of the high association between diabetes, obesity, and hypertension, and the increase in risk of heart attack in patients exhibiting both diabetes and hypertension (see, e.g., Tzagournis, Am. J. Med., 86 (suppl 1B):50-54 (1989)), what is needed in the art is an agent arch will treat both diabetes and hypertension.

Thiazolidine derivatives useful for the treatment of diabetes are described in U.S. patents 4,287,200; 4,687,777; and 4,572,912. Their effect on insulin resistance are described, e.g., Chang, et al, Diabetes 32:839-845 (1983) and Chang, et al, Diabetes 32:830-838 (1983). The association between circulating insulin and hypertension has been discussed in the literature, as described above.

The present invention provides for treating or preventing hypertension in an insulin-resistant patient who is not exhibiting diabetes, using an insulin sensitizing compound selected from thiazolidinediones (see formula in claim 1) such as ciglitazone, pioglitazone hydrochloride, CS 045, metformin, antidiabetic indole amines and thermogenic beta agonists.

Surprisingly and unexpectedly, the present invention provides a class of agents useful to treat insulin resistant patients; these agents have an especially good effect in the lowering of blood pressure in said patients.

By insulin sensitizing agent is meant any agent which will lower blood glucose levels by increasing the responsiveness of the tissues to insulin.

Patients susceptible to insulin resistant hypertension exhibit insulin resistance and are therefore likely to exhibit hypertension. Such patients are well known and readily determinable by a physician of ordinary skill in the art.

By treatment is meant any lowering of blood pressure caused by insulin resistance and/or high circulating insulin levels. By prevention is meant partial to total avoidance of hypertension in insulin resistant patients, depending on the severity of the disease.

The thiazolidinediones are particularly useful in the present invention and are made by the methods described in U.S. patents 4,287,200; 4,687,777; and 4,572,912. The dosage forms and modes of administration described therein are also useful for carrying out the method of the present invention. More specific dose ranges are set out below.

Thermogenic beta agonists are a well known class of antidiabetic agents, exemplified by, e.g., compounds BRL 26,830 (see Biochemica and Biophysica Research Comm. 128:928-935 (1985); and BRL 35,135 (Diabetes, Vol. 35: Abstract No. 262 and 263, 1986) being developed by SmithKline-Beecham. Metformin is described, e.g., in Petersen, et al., Diabetic Medicine 6:249-256 (1989). A class of diabetic indole amines are described in EP-A-0369533 and WO-A-90/05721.

The preferred compounds of this invention include ciglitazone, (2,4-thiazolidinedione, 5-[[4-[(1-methylcyclohexyl)methoxy]phenyl]methyl]-, (±)- or (±)-5-[*p*-[(1-methylcyclohexyl)methoxy]benzyl]-2,4-thiazolidinedione);Pioglitazonehydrochloride(5-[[4-[2-(5-ethyl-2-pyridinyl-ethoxy]phenyl]methyl]-,monohydrochloride, (±);(2)(±)-5-[*p*-[2-(5-ethyl-2-pyridyl)ethoxy]benzyl]-2,4-thiazolidinedione monohydrochloride) ; and CS 045 (5-(4-((3,4-dihydro-6-hydroxy-2,5,7,8 tetramethyl-2H-1-benzopyran-2-yl)methoxy)phenyl)methyl)-2,4-thiazolidinedione).

While any convenient route of administration is employed, the preferred thiazolidinedione compounds of the present invention are preferably orally administered to humans to affect insulin sensitization for the purpose of favorably affecting blood pressure. For this purpose, the compounds are administered from 100 micrograms per kg to 6 mg per kg per dose, administered from 1 to 3 times daily. Other routes of administration, such as parenteral (including intravenous, intramuscular, and intraperitoneal) are also employed. Equipotent doses for the other compounds of this invention and the other routes of administration would thus be employed, and could be readily determined by a physician of ordinary skill.

The exact dose depends on the age, weight, and condition of the patient and the frequency and route of administration. Such variations are within the skill of the practitioner or can readily be determined.

The employment of sound medical therapy requires that the compounds of this invention be employed prophylactically only in cases where the animal or patient is particularly susceptible to the development of hypertension. The conditions and circumstances which increase the susceptibility are readily ascertainable to the ordinary skilled physician and include glucose intolerance, insulin resistance, hyperinsulinemia and obesity.

In the prophylactic use of these compounds, the dose effective for the prevention of hypertension is readily determined by patient response, as discussed above for therapeutic uses, and is, in general, somewhat less than the dose required to treat the disease.

The present invention is seen more fully by the Example given below.

### Example 1

Ciglitazone was tested in the Zucker rat, a well known model of insulin resistant mammals and was shown to lower blood pressure, as described below:

Two groups of 6-week old obese female Zucker (fa/fa) rats, 10 control and 10 experimental, were fed a diet containing: 65 % carbohydrate, 18 % protein, 5 % fat, 5 % fiber, 0.1 % sodium chloride (NaCl), with the remainder containing water, vitamins and minerals.

The experimental group received the drug (ciglitazone powder) as a 0.05 % (w/w) dietary admixture (33 to 58 mg/kg body weight/day, calculated from food intake) for 30 days. The control group did not receive the drug.

The mean arterial pressure (MAP) was measured in the unanesthetized, unrestrained state by indwelling femoral artery catheters attached to a pressure transducer, and blood drawn for measurement of blood glucose and plasma insulin concentrations. The results of the study are set forth in Table 1.

### Example 2

The effect of insulin sensitizing compounds in primates was shown as follows. Obese, insulin-resistant Rhesus monkeys were given pioglitazone (1 mg/kg/day, oral gavage) for two weeks. Glucose tolerance was substantially improved in 5 of 6 monkeys. Systolic blood pressure was reduced an average of 16 mmHg; mean arterial blood pressure (MAP) was reduced an average of 8.4 mmHg. These data show that improved insulin sensitivity produced by drugs of this type are an effective treatment for lowering blood pressure.

**TABLE 1**

| Effects of Ciglitazone on Mean Arterial Pressure (MAP) and Urine Output | | | |
|---|---|---|---|
| Measurement | Control | Ciglitazone | Significance¹ |
| MAP (mm Hg) | 119 ± 2 (n=9) | 112 ± 4 (n=6) | p <0.05² |
| Urine Output | 80 ± 5 (n=9) | 97 ± 8 (n=6) | p <0.05³ |
| Insulin (mU/ml) | 171 ± 20 | 60 ± 9 | |

| | | | |
|---|---|---|---|
| 1. The data is presented as the mean ± SEM and significance determined with the paired Students' t-test. | | | |
| 2. The one-tailed t-test was used to compare blood pressure measurements. | | | |
| 3. The two-tailed t-test was used to compare urine output measurements. | | | |

There was no significant difference in body weight or food intake between both groups over the period of the experiment. Because of complications during surgery, one animal was lost from the control group, and 4 from the experimental group.

Ciglitazone significantly lowered blood pressure in the fa/fa Zucker rats.

## Claims

1. Use of an insulin-sensitising compound to prepare a medicament for treating or preventing hypertension in an insulin-resistant patient who is not exhibiting diabetes, wherein the compound is selected from pioglitazone hydrochloride, metformin, antidiabetic indole amines, thermogenic β-agonists, CS 045 and thiazolidinedione derivatives of the general formula 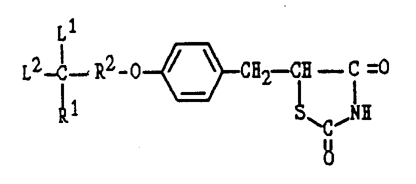 wherein R¹ is alkyl of 1 to 10 carbon atoms, cycloalkyl of 3 to 7 carbon atoms, phenylalkyl of 7 to 11 carbon atoms, or phenyl; R² is a bond or a lower alkylene group; and L¹ and L² are the same or different and are each lower alkyl or L¹ and L² are combined to form an alkylene group, provided that when R¹ is other than alkyl, L¹ and L² may further be hydrogen.

2. A use of claim 1, wherein the compound is a thiazolidinedione as defined therein.

3. A use of claim 2, wherein the compound is ciglitazone.

4. A use of claim 1, wherein the compound is pioglitazone hydrochloride.

5. A use of claim 1, wherein the compound is metformin.

6. A use of claim 1, wherein the compound is an antidiabetic indole amine.

7. A use of claim 1, wherein the compound is a thermogenic β-agonist.

8. A use of claim 1, wherein the compound is CS 045.

## Patentansprüche

1. Verwendung einer insulinsensibilisierenden Verbindung zur Herstellung eines Medikaments zur Behandlung oder Verhinderung von Bluthochdruck bei einem insulinresistenten Patienen, der kein Diabetiker ist, wobei die Verbindung aus Pioglitazone-Hydrochlorid, Metformin, antidiabetischen Indolaminen, thermogenen β-Agonisten, CS 045 und Thiazolidindion-Derivaten der allgemeinen Formel I 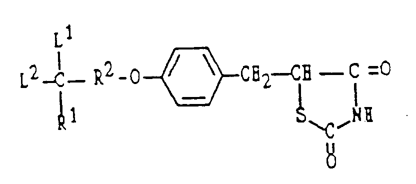 worin bedeuten:
R¹ Alkyl mit 1 bis 10 Kohlenstoffatom(en), Cycloalkyl mit 3 bis 7 Kohlenstoffatomen, Phenylalkyl mit 7 bis 11 Kohlenstoffatomen oder Phenyl;
R² eine Bindung oder eine Niedrigalkylengruppe;
L¹ und L², die gleich oder verschieden sein können, jeweils Niedrigalkyl oder
L¹ und L² zusammen eine Alkylengruppe, wobei gilt, dass im Falle, dass R¹ von Alkyl verschieden ist, L¹ und L² auch für Wasserstoff stehen können,
ausgewählt ist.

2. Verwendung nach Anspruch 1, wobei die Verbindung aus einem Thiazolidindion der angegebenen Definition besteht.

3. Verwendung nach Anspruch 2, wobei es sich bei der Verbindung um Ciglitazone handelt.

4. Verwendung nach Anspruch 1, wobei es sich bei der Verbindung um Pioglitazone-Hydrochlorid handelt.

5. Verwendung nach Anspruch 1, wobei es sich bei der Verbindung um Metformin handelt.

6. Verwendung nach Anspruch 1, wobei es sich bei der Verbindung um ein antidiabetisches Indolamin handelt.

7. Verwendung nach Anspruch 1, wobei es sich bei der Verbindung um einen thermogenen β-Agonisten handelt.

8. Verwendung nach Anspruch 1, wobei es sich bei der Verbindung um CS 045 handelt.

## Revendications

1. Utilisation d'un composé de sensibilisation à l'insuline pour préparer un médicament destiné au traitement ou à la prévention de l'hypertension chez un patient résistant à l'insuline qui ne présente pas de diabète, dans laquelle le composé est choisi entre le chlorhydrate de pioglitazone, la metformine, des indolamines antidiabétiques, des agonistes β-thermogènes, le CS 045 et des dérivés de thiazolidinedione de formule générale 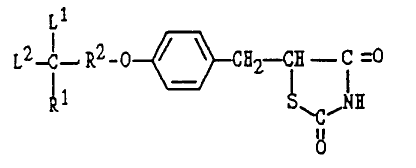 dans laquelle R¹ représente un groupe alkyle ayant 1 à 10 atomes de carbone, cycloalkyle ayant 3 à 7 atomes de carbone, phénylalkyle ayant 7 à 11 atomes de carbone ou phényle ; R² représente une liaison ou un groupe alkylène inférieur ; et L¹ et L² sont identiques ou différents et représentent chacun un groupe alkyle inférieur ou bien L¹ et L² sont associés pour former un groupe alkylène ; sous réserve que, lorsque R¹ est autre qu'un groupe alkyle, L¹ et L² puissent représenter en outre l'hydrogène.

2. Utilisation suivant la revendication 1, dans laquelle le composé est une thiazolidinedione répondant à la définition figurant dans le présent mémoire.

3. Utilisation suivant la revendication 2, dans laquelle le composé est la ciglitazone.

4. Utilisation suivant la revendication 1, dans laquelle le composé est le chlorhydrate de la pioglitazone.

5. Utilisation suivant la revendication 1, dans laquelle le composé est la metformine.

6. Utilisation suivant la revendication 1, dans laquelle le composé est une indolamine antidiabétique.

7. Utilisation suivant la revendication 1, dans laquelle le composé est un agoniste β-thermogène.

8. Utilisation suivant la revendication 1, dans laquelle le composé est le CS 045.
